# EUROPEAN PATENT SPECIFICATION

(11) **EP 3 682 908 B1**
(45) Date of publication and mention of the grant of the patent: **11.12.2024**
(21) Application number: 19152857.9
(22) Date of filing: 21.01.2019
(51) Int. Cl.: A61L 2/10, B29C 45/14, E05B 1/00, F21V 8/00, G02B 6/10, H05B 33/12, F21K 9/61

(54) **ANTIBACTERIAL FILM COMPONENT**
ANTIBAKTERIELLE FOLIENKOMPONENTE
COMPOSANT DE FILM ANTIBACTÉRIEN

(43) Date of publication of application: 22.07.2020
(73) Proprietor: ABB Schweiz AG, 5400 Baden (CH)
(72) Inventor: MARTINI, Harald, 722 18 Västerås (SE); THOLENCE, Frédéric, 723 48 Västerås (SE); ZHAO, Su, 723 56 Västerås (SE)
(74) Representative: Kransell & Wennborg KB

(56) References cited:
- DE-A1- 102009 059 219
- US-A1- 2009 129 107
- GEORG H KUHLMANN ET AL: "Chapter 4: Manufacuring of Composites Melt Compression Molding (MCM) a One-shot Process for In-mold Lamination and Compression Molding by Melt Strip Deposition Special Molding Techniques LOW PRESSURE INJECTION MOULDING TECHNICS", SPECIALIZED MOLDING TECHNIQUES : APPLICATION, DESIGN, MATERIALS AND PROCESSING, 31 December 2002 (2002-12-31), pages 171 - 181, XP055610481, ISBN: 978-1-884207-91-4, Retrieved from the Internet <URL:https://ebookcentral.proquest.com/lib/epo-ebooks/reader.action?docID=421038&ppg=182> [retrieved on 20190801]

## Description

### TECHNICAL FIELD

The present disclosure relates to components comprising an anti-bacterial film, e.g. light switches, door handles or the like, and to a method of producing such a component.

### BACKGROUND

Multi resistant bacteria are becoming more and more a problem, especially in hospital environment. To minimize the risk of contamination, extensive cleaning of surfaces which are touched by many people should be performed frequently (which is time consuming and costly). Nowadays, to obtain a surface with anti-microbial function, additional coatings (based on toxic or metallic particles) are sometimes applied, or anti-microbial additives are added to the resin of e.g. light switches.

Hospital-acquired infections can be caught by direct contact with common surfaces like switches, door handles etc. To break the cycle of contamination, an anti-microbial finishing can be provided for such surfaces. The anti-microbial effect is obtained either by applying an anti-microbial coating or anti-bacterial additives into the plastic resin. In both cases toxic metal ions (e.g. Ag or Cu) or other chemicals are slowly released into the environment resulting in a limited lifetime of the biocidal effect as well as contamination of the environment. The coating may be difficult to apply (additional process step) and costly. Abrasion of the coating will also reduce the anti-microbial function.

Ultraviolet (UV) light, as well as visible violet and blue light, is known to have bactericidal or inhibiting effects. For instance, rooms may be irradiated with UV light when not in use. However, when in use, such general irradiation would be a health hazard for persons in the room.

CN 107170 601 discloses the use of an ultraviolet lamp in a light switch such that the transparent button of the switch is disinfected by the ultraviolet light.

US 8,226,255 discloses a key button with an anti-bacterial light source. The button has a transparent keycap. Ultraviolet light emitted by an ultraviolet lighting element enter into a light guide block, and is then reflected by the light guide block to pass through the keycap to arrive to a second side surface of the keycap.

DE 10 2009 059219 discloses e.g. a front panel of a drawer, guiding disinfectant UV light.

US 2009/129107 discloses a moulded decorative part for a vehicle interior, comprising an excitation source for emitting electromagnetic waves and a luminescent material which is excitable by the electromagnetic waves from the excitation source to emit light in the visible range.

### SUMMARY

The inventors have now realised that an in-mould decoration/back injection moulding process can be used to combine a light emitting film (e.g. comprising a light source or being a light guide for an external light source) with a moulded substrate, e.g. a part of a light switch or door handle or the like which is often touched by different persons, for anti-microbial (e.g. antibacterial) function using blue light. In-mould decoration makes it possible to obtain the anti-microbial function during moulding of the component (i.e. substrate plus film) in a cost efficient way. No additional coating step may be necessary. No release of toxic particles will occur either from a coating or from the component itself.

The present invention is defined by the appended claims.

It is to be noted that any feature of any of the aspects may be applied to any other aspect, wherever appropriate. Likewise, any advantage of any of the aspects may apply to any of the other aspects. Other objectives, features and advantages of the enclosed embodiments will be apparent from the following detailed disclosure, from the attached dependent claims as well as from the drawings.

Generally, all terms used in the claims are to be interpreted according to their ordinary meaning in the technical field, unless explicitly defined otherwise herein. All references to "a/an/the element, apparatus, component, means, step, etc." are to be interpreted openly as referring to at least one instance of the element, apparatus, component, means, step, etc., unless explicitly stated otherwise. The steps of any method disclosed herein do not have to be performed in the exact order disclosed, unless explicitly stated. The use of "first", "second" etc. for different features/components of the present disclosure are only intended to distinguish the features/components from other similar features/components and not to impart any order or hierarchy to the features/components.

### BRIEF DESCRIPTION OF THE DRAWINGS

Embodiments will be described, by way of example, with reference to the accompanying drawings, in which:
Fig 1a is a schematic sectional side view of a mould where an antibacterial film is applied to a movable mould half, in accordance with embodiments of the present invention.
Fig 1b is a schematic sectional side view of the mould of figure 1a, where the mould has been closed, forming a mould cavity between the movable and fixed halves of the mould, in accordance with embodiments of the present invention.
Fig 2 is a schematic side view of a component comprising an antibacterial film on a moulded substrate, e.g. with the film acting as a light guide for a light emitted from a light source which is external to the film, in accordance with embodiments of the present invention.
Fig 3 is a schematic exploded view of an antibacterial film comprising a plurality of layers, e.g. a light-emitting film by electroluminescence, in accordance with embodiments of the present invention.
Fig 4 is a schematic flow chart of a method of producing a moulded component, in accordance with embodiments of the present invention.

### DETAILED DESCRIPTION

Embodiments will now be described more fully hereinafter with reference to the accompanying drawings, in which certain embodiments are shown. However, other embodiments in many different forms are possible within the scope of the present disclosure. Rather, the following embodiments are provided by way of example so that this disclosure will be thorough and complete, and will fully convey the scope of the disclosure to those skilled in the art. Like numbers refer to like elements throughout the description.

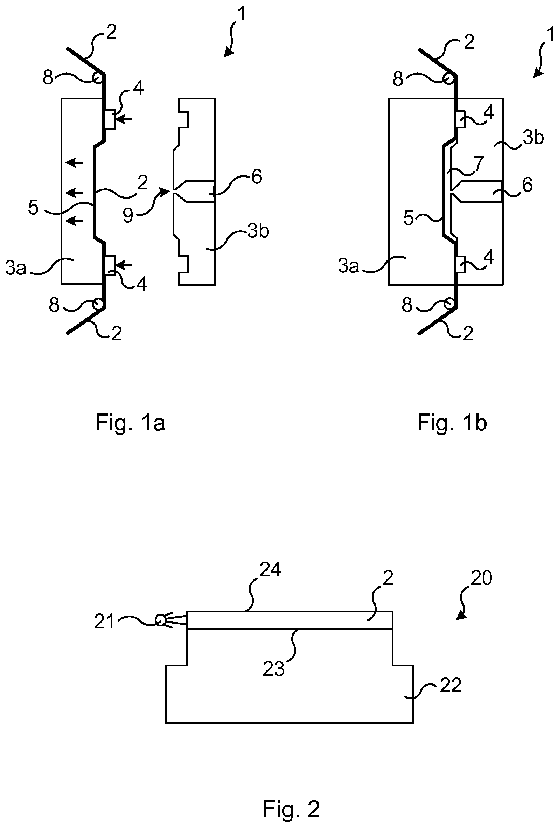

Figures 1a and 1b illustrate a mould 1 which can be used for so called in-mould decoration and/or back injection moulding to combine a flexible film 2 (for emitting the antibacterial light) with the plastic substrate to form a component (part of switch, button, handle or the like), to obtain an anti-microbial surface of the component. The mould typically comprises a movable mould part (movable mould half), which is shown to the left in the figures, and a fixed mould part (fixed mould half), which is shown to the right in the figures.

Figure 1a shows the mould 1 open, with a movable half 3a and a fixed half 3b of the mould separated from each other. The film 2 maybe introduced into the mould 1 as a web being passed between the movable and fixed halves 3a and 3b e.g. by means of rolls 8. The film 2 is applied to an inner surface 5 of the movable half 3a, e.g. by means of clamps 4 and/or suction as illustrated by the arrows in the figure.

Figure 1b shows the mould being closed, by the movable half 3a being moved towards the fixed half 3b such that the fixed half 3b is pressed against the movable half 3a, forming a mould cavity 7 there between. The inner surface 5 of the movable half 3a, having the film 2 applied to at least a part thereof, forms a wall delimiting the mould cavity 7. When the mould is thus closed, the plastic substrate material can be injected into the mould cavity 7, typically via a hole (called spruce) 6 having an opening gate 9 into the mould cavity.

When the material is injected in liquid, e.g. molten, form, the material fills the mould cavity 7 and is formed into the substrate onto a top surface of which the film 2 is adhered. If the substrate material is thermoplastic, the material is injected at an elevated temperature at which the material is liquid. The material is then cooled within the mould cavity whereby it is solidified. Similarly, if the substrate material is curable, the material may be cured within the mould cavity whereby it is solidified.

The solid substrate having the film 2 adhered to a top surface thereof may then be removed from the mould 1, after the movable and fixed halves 3a and 3b have once again been separated from each other.

Figure 2 illustrates a component 20 comprising a moulded substrate 22 having a film 2 adhered to a top surface 23 thereof, which may have been produced in accordance with the injection moulding process discussed in relation to figures 1a and 1b. That the surface 23 to which the film 2 is adhered is a top surface implies that it is an outer surface which is arranged to be proximal to touches from persons using the component. The film is thus arranged to be touched by such persons, which is why it is desirable that the film has antibacterial (or, more generally, anti-microbial) properties to avoid transmission of pathogens from one person to another.

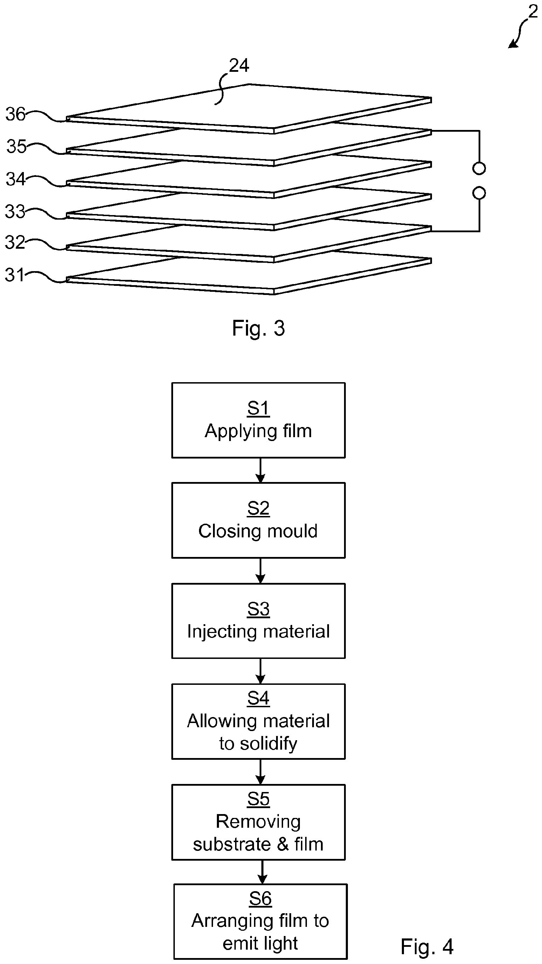

In accordance with the present invention, the film is arranged to emit light having antibacterial properties from a light source. As discussed in relation to figure 3, the light source may be comprised in the film 2. However, in the embodiment of figure 2, the light source 21 is external to the film 2 and the film is arranged to act as a light guide, guiding light from the external light source and emitting it from and throughout its top surface 24. Thus, bacteria and other microbes deposited on the top surface 24 of the film (typically by a person touching the film) may be inhibited or killed by the light emitted there from. In order to act as a light guide, the film 2 is transparent, or comprises at least one transparent top layer, e.g. of polycarbonate (PC) or poly(methyl methacrylate) (PMMA) material.

The light source 21 may be or comprise any light source emitting sufficient amounts of light of a suitable wavelength to be antibacterial. It may be preferred to use a light source which specifically emits light at such a suitable wavelength, thus emitting in a narrow spectrum. Examples of such light sources include a Light-Emitting Diode (LED) and a laser. An LED may be preferred since it is relatively cheap and easily maintained, and may give a relatively uniform emission of light over the top surface 24 of the film. The light source 21 may comprise a plurality (e.g. array) of LEDs or other light sources, e.g. arranged along a side of the film 2. The LED may e.g. comprise indium gallium nitride (InGaN) which typically gives blue or violet visible light, but any other suitable LED type may alternatively be used.

Figure 3 illustrates that the film 2 may be composed of a plurality of different layers, typically laminated to each other. If the light source is comprised in the film 2, the film may comprise a luminous layer 34, e.g. an electroluminescent layer or a fluorescent layer, emitting light at an antibacterial wavelength.

For instance, if the luminous layer 34 is electroluminescent, the luminous layer 34 is typically stacked between a back electrode layer 32 and a front electrode layer 35. A dielectric layer 33 may be included between the back electrode layer 32 and the luminous layer 34. An electrically insulating bottom layer 31 maybe included in the film to insulate the electrode layers from the substrate 22. Similarly, an electrically insulating top layer 36 may be included in the film to insulate the electrodes from e.g. persons touching the film 2, e.g. of polycarbonate (PC) or poly(methyl methacrylate) (PMMA) material. When a voltage is applied between the back and front electrode layers 32 and 35, the luminous layer 34 will be induced to emit light of a desired antibacterial wavelength. For the emitted light to be emitted through the top surface 24 of the film, any layers 35 and 36 positioned above the luminous layer 34 (i.e. between the luminous layer 34 and the top surface 24 of the film) are transparent to that light.

Regardless of whether the light source is external to or comprised in the film 2, and regardless of whether the light source comprises an array of distinct light sources, the light source is arranged to emit visible light having a wavelength within the range of 400 nm to 470 nm, e.g. 430 nm to 460 nm, which is easily obtained e.g. by means of LED while not being harmful to humans as UV light of shorter wavelengths may be.

Figure 4 is a schematic flow chart of an embodiment of the method of producing the moulded component 20, of the present disclosure. The method comprises applying S1 a film 2 to an inner wall 5 of a movable half 3a of the mould 1. The method also comprises closing S2 the mould 1 such that a mould cavity 7 is formed between the inner wall 5, having the film 2 applied there to, and a fixed half 3b of the mould. The method also comprises injecting S3 a plastic substrate material into the cavity 7 through a spruce 6 in the mould 1, such that a substrate 22 is formed in the cavity, the film 2 adhering to a top surface 23 of said substrate 22. The method also comprises allowing the substrate material to solidify. The method also comprises removing S5 the substrate 22 and thereto adhered film 2 from the mould 1. The method also comprises arranging S6 the film 2 to emit light having antibacterial properties from a light source 21 and/or 34 forming the component 20 with said substrate 22 and thereto adhered film 2.

The light source is arranged to emit visible light having a wavelength within the range of 400 nm to 470 nm, e.g. 430 nm to 460 nm. These wavelength have been shown to have anti-microbial properties. Visible light may be preferred in order to avoid any health concerns relating to UV light.

In some embodiments of the present invention, the film 2 is configured to act as a light guide for light emitted from the light source 21 when it is located externally of the film, e.g. as in figure 2. In some embodiments, the light source 21 comprises a Light-Emitting Diode (LED) e.g. comprising indium gallium nitride (InGaN) which is associated with suitable wavelengths.

Additionally or alternatively, in some embodiments of the present invention, the light source is comprised in the film 2, e.g. in the form of a luminous layer 34 of the film 2. In some embodiments, the luminous layer 34 is an electroluminescent layer. Alternatively, the luminous layer 34 may be a fluorescent layer.

In some embodiments of the present invention, the component 20 is, or is part of, a light switch or a door handle, especially a light switch, which are examples of components which are typically touched by different persons in e.g. a hospital or laboratory environment.

The present disclosure has mainly been described above with reference to a few embodiments. However, as is readily appreciated by a person skilled in the art, other embodiments than the ones disclosed above are equally possible within the scope of the present disclosure, as defined by the appended claims.

## Claims

1. A component (20) comprising:
a substrate (22) of a plastic material; and
an antibacterial flexible film (2) adhered to a top surface (23) of the substrate by a moulding process;
wherein the component further comprises a light source (21/34) such that the film is arranged to emit light having antibacterial properties from said light source;
**characterized in that**
the light source is arranged to emit visible light having a wavelength within the range of 400 nm to 470 nm.

2. The component of claim 1, wherein the light source is arranged to emit visible light having a wavelength within the range of 430 nm to 460 nm.

3. The component of any preceding claim, wherein the film (2) is configured to act as a light guide for light emitted from the light source (21) located externally of the film.

4. The component of claim 3, wherein the light source (21) comprises a Light-Emitting Diode, LED, e.g. comprising indium gallium nitride, InGaN.

5. The component of any preceding claim, wherein the light source (34) is comprised in the film (2).

6. The component of claim 5, wherein the light source comprises a luminous layer (34) of the film (2).

7. The component of claim 6, wherein the luminous layer (34) is an electroluminescent layer.

8. The component of any preceding claim, wherein the component (20) is, or is part of, a light switch or a door handle, especially a light switch.

9. A method of producing a component (20), the method comprising:
applying (S1) a film (2) to an inner wall (5) of a movable half (3a) of a mould (1);
closing (S2) the mould (1) such that a mould cavity (7) is formed between the inner wall (5), having the film (2) applied there to, and a fixed half (3b) of the mould;
injecting (S3) a plastic substrate material into the cavity (7) through a spruce (6) in the mould (1), such that a substrate (22) is formed in the cavity, the film (2) adhering to a top surface (23) of said substrate;
allowing (S4) the substrate material to solidify;
removing (S5) the substrate (22) and thereto adhered film (2) from the mould (1); and
by means of the film (2) being configured to act as a light guide for light emitted from an external light source (21) or by means of a light source (34) comprised in the film (2), arranging (S6) the film (2) to emit light having antibacterial properties from a light source (21/34) forming the component (20) with said substrate (22) and thereto adhered film (2),
**characterized in that**
the light source is arranged to emit visible light having a wavelength within the range of 400 nm to 470 nm.

## Patentansprüche

1. Komponente (20) umfassend:
ein Substrat (22) aus einem Kunststoffmaterial; und
einen antibakteriellen flexiblen Film (2), der durch ein Formverfahren an eine obere Oberfläche (23) des Substrats gehaftet ist;
wobei die Komponente ferner eine Lichtquelle (21/34) umfasst, wobei der Film dafür beschaffen ist, Licht mit antibakteriellen Eigenschaften aus der Lichtquelle zu emittieren;
**dadurch gekennzeichnet, dass**
die Lichtquelle dafür beschaffen ist, sichtbares Licht mit einer Wellenlänge in dem Bereich von 400 nm bis 470 nm zu emittieren.

2. Komponente nach Anspruch 1, wobei die Lichtquelle dafür beschaffen ist, sichtbares Licht mit einer Wellenlänge in dem Bereich von 430 nm bis 460 nm zu emittieren.

3. Komponente nach einem der vorstehenden Ansprüche, wobei der Film (2) dafür gestaltet ist, als Lichtleiter für Licht zu wirken, das von der Lichtquelle (21), die außerhalb des Films angeordnet ist, emittiert wird.

4. Komponente nach Anspruch 3, wobei die Lichtquelle (21) eine lichtemittierende Diode, LED, umfasst, die z.B. Indium-Galliumnitrid, InGaN, umfasst.

5. Komponente nach einem der vorstehenden Ansprüche, wobei die Lichtquelle (34) in dem Film (2) enthalten ist.

6. Komponente nach Anspruch 5, wobei die Lichtquelle eine Leuchtschicht (34) des Films (2) umfasst.

7. Komponente nach Anspruch 6, wobei die Leuchtschicht (34) eine Elektrolumineszenzschicht ist.

8. Komponente nach einem der vorstehenden Ansprüche, wobei die Komponente (20) ein Lichtschalter oder ein Türgriff, insbesondere ein Lichtschalter, ist oder Teil davon ist.

9. Verfahren zur Herstellung einer Komponente (20), wobei das Verfahren umfasst:
Aufbringen (S1) eines Films (2) auf eine Innenwand (5) einer beweglichen Hälfte (3a) eines Formwerkzeugs (1);
Schließen (S2) des Formwerkzeugs (1), so dass ein Formhohlraum (7) zwischen der Innenwand (5), auf der der Film (2) aufgebracht ist, und einer feststehenden Hälfte (3b) des Formwerkzeugs gebildet wird;
Einspritzen (S3) eines Kunststoffsubstratmaterials in den Hohlraum (7) durch einen Einguss (6) in dem Formwerkzeug (1), so dass ein Substrat (22) in dem Hohlraum gebildet wird, wobei der Film (2) an einer oberen Oberfläche (23) des Substrats haftet;
Erlauben (S4), dass das Substratmaterial erstarrt;
Entnehmen (S5) des Substrats (22) und des daran haftenden Films (2) aus dem Formwerkzeug (1); und
mithilfe des Films (2), der dafür ausgelegt ist, als Lichtleiter für von einer externen Lichtquelle (21) emittiertes Licht zu wirken, oder mithilfe einer Lichtquelle (34), die in dem Film (2) enthalten ist, Anordnen (S6) des Films (2), um Licht mit antibakteriellen Eigenschaften von einer Lichtquelle (21/34) zu emittieren, um die Komponente (20) mit dem Substrat (22) und dem daran haftenden Film (2) zu bilden, **dadurch gekennzeichnet, dass**
die Lichtquelle dafür beschaffen ist, sichtbares Licht mit einer Wellenlänge in dem Bereich von 400 nm bis 470 nm zu emittieren.

## Revendications

1. Composant (20) comprenant :
un substrat (22) dans un matériau plastique ; et
un film souple antibactérien (2) collé à une surface supérieure (23) du substrat par un procédé de moulage ;
le composant comprenant en outre une source de lumière (21/34) de telle sorte que le film est agencé pour émettre de la lumière ayant des propriétés antibactériennes depuis ladite source de lumière ;
**caractérisé en ce que**
la source de lumière est agencée pour émettre de la lumière visible ayant une longueur d'onde dans la plage de 400 nm à 470 nm.

2. Composant selon la revendication 1, dans lequel la source de lumière est agencée pour émettre de la lumière visible ayant une longueur d'onde dans la plage de 430 nm à 460 nm.

3. Composant selon une quelconque revendication précédente, dans lequel le film (2) est conçu pour agir comme guide de lumière pour la lumière émise depuis la source de lumière (21) située à l'extérieur du film.

4. Composant selon la revendication 3, dans lequel la source de lumière (21) comprend une diode électroluminescente, LED, comprenant par ex. du nitrure d'indium et de gallium, InGaN.

5. Composant selon une quelconque revendication précédente, dans lequel la source de lumière (34) est comprise dans le film (2).

6. Composant selon la revendication 5, dans lequel la source de lumière comprend une couche lumineuse (34) du film (2).

7. Composant selon la revendication 6, dans lequel la couche lumineuse (34) est une couche électroluminescente.

8. Composant selon une quelconque revendication précédente, le composant (20) étant ou faisant partie d'un interrupteur d'éclairage ou d'une poignée de porte, en particulier d'un interrupteur d'éclairage.

9. Procédé de production d'un composant (20), le procédé comprenant :
l'application (S1) d'un film (2) à une paroi interne (5) d'une moitié mobile (3a) d'un moule (1) ;
la fermeture (S2) du moule (1), de telle sorte qu'une cavité de moule (7) est formée entre la paroi interne (5), avec le film (2) appliqué à celle-ci, et une moitié fixe (3b) du moule ;
l'injection (S3) d'un matériau substrat plastique à l'intérieur de la cavité (7) par un trou (6) dans le moule (1), de telle sorte qu'un substrat (22) est formé dans la cavité, le film (2) collant à une surface supérieure (23) dudit substrat ;
l'opération consistant à laisser (S4) le matériau substrat solidifier ;
le retrait (S5) du substrat (22) et du film (2) collé à celui-ci du moule (1) ; et
au moyen du film (2) conçu pour agir comme guide de lumière pour la lumière émise depuis une source de lumière externe (21) ou au moyen d'une source de lumière (34) comprise dans le film (2), l'agencement (S6) du film (2) pour émettre de la lumière ayant des propriétés antibactériennes depuis une source de lumière (21/34) formant le composant (20) avec ledit substrat (22) et ledit film (2) collé à celui-ci,
**caractérisé en ce que**
la source de lumière est agencée pour émettre de la lumière visible ayant une longueur d'onde dans la plage de 400 nm à 470 nm.
